# EUROPEAN PATENT APPLICATION

(11) **EP 3 943 603 A1**
(43) Date of publication of application: **26.01.2022**
(21) Application number: 20187230.6
(22) Date of filing: 22.07.2020
(51) Int. Cl.: C12N 15/63, C12N 15/75, C12Q 1/68

(54) **RAPID TRANSFORMATION METHOD FOR GRAM-POSITIVE BACTERIA**

(71) Applicant: Technische Universität Darmstadt, 64289 Darmstadt (DE)
(72) Inventor: Kabisch, Johannes, 64367 Mühltal (DE); Schlichting, Niels, 64283 Darmstadt (DE); Karava, Marianna, 64285 Darmstadt (DE)
(74) Representative: Fuchs Patentanwälte Partnerschaft mbB

(57) **Abstract**

A method is described, which enables a simplified and faster one-step transformation of Gram-positive bacteria. Uses of that method include the transient and stable expression of a plurality of copies of a gene of interest (GOI) in a defined gene locus of Gram-positive bacteria. Gram-positive bacteria generated by the new transformation method are also described.

SEQUENCE LISTING

<110>  Technische Universität Darmstadt, Karolinenpl. 5, 64289 Darmstadt

<120>  Rapid transformation method for Gram-positive bacteria

<130>  //App File Ref//

<140>  //Appl No//
<141>  2020-07-21

<160>  8

<170>  PatentIn version 3.5

<210>  1
<211>  20
<212>  DNA
<213>  Artificial Sequence

<220>
<223>  GFPmut2, amplicon 1, 10453-fw

<400>  1
tgttccatgg ccaacacttg                                                    20


<210>  2
<211>  24
<212>  DNA
<213>  Artificial Sequence

<220>
<223>  GFPmut2, amplicon 1, 10454-rv

<400>  2
tcatgctgtt tcatatgatc tggg                                               24


<210>  3
<211>  25
<212>  DNA
<213>  Artificial Sequence

<220>
<223>  GFPmut2, amplicon 2, 10455-fw

<400>  3
tcacacaatg tatacatcat ggcag                                              25


<210>  4
<211>  24
<212>  DNA
<213>  Artificial Sequence

<220>
<223>  GFPmut2, amplicon 2, 10456-rv

<400>  4
aacgcttcca tcttcaatgt tgtg                                               24

<210>  5
<211>  21
<212>  DNA
<213>  Artificial Sequence

<220>
<223>  MKate 2, amplicon 1,  10459-fw

<400>  5
atggtgagcg agctgattaa g                                                21


<210>  6
<211>  20
<212>  DNA
<213>  Artificial Sequence

<220>
<223>  MKate 2, amplicon 1,  10460-rv

<400>  6
gattctcatg gtctgggtgc                                                  20


<210>  7
<211>  24
<212>  DNA
<213>  Artificial Sequence

<220>
<223>  MKate 2, amplicon 2, 10461-fw

<400>  7
aacttgaaga ccacatacag atcc                                             24


<210>  8
<211>  20
<212>  DNA
<213>  Artificial Sequence

<220>
<223>  MKate 2, amplicon 2, 10462-rv

<400>  8
tgttccatgg ccaacacttg                                                  20

## Description

A method is described, which enables a simplified and faster "single-batch" transformation of Gram-positive bacteria. Uses of that method include the transient and stable expression of a plurality of copies of a gene of interest (GOI) in a defined gene locus of Gram-positive bacteria. Gram-positive bacteria generated by the new transformation method are also described.

### Background of the Invention

Gram-positive bacteria, such as the bacterium *Bacillus subtilis,* can be transformed to microbial cell factories by genetic programming. As such they can produce valuable molecules in an efficient and renewable manner.

For this approach, recombinant DNA needs to be either expressed transiently or stably in the host organism (i.e. integrated into the host's chromosome).

However, the thick peptidoglycan layer in Gram-positive bacteria's cell wall prevents the use of the rather simple transformation techniques developed for Gram-negative bacteria.

Due to lower transformation efficiencies of Gram-positive bacteria with conventional methods, the amplification of newly constructed expression vectors is performed using the Gram-negative bacterium *E. coli* as a shuttle host. This in turn results in increased development times since the shuttling (transformation, verification and isolation of the assembled plasmid) takes at least two days.

The same holds true for plasmids delivered from gene synthesis companies. Usually they first have to be shuttled through *E. coli* in order to obtain sufficient DNA-yields for transformation into Gram-positive bacteria, such as the bacterium *Bacillus subtilis.*

If the expression construct of interest is toxic for *E. coli* the shuttling process proves difficult or impossible.

Further, the transformation of replicative plasmids into Gram-positive bacteria, such as the bacterium *Bacillus subtilis,* requires a multimeric form and the cell-wall needs to be weakened by chemical pretreatments with polyethylene glycol or glycine. Alternatively, the generation of protoblasts through lysozyme treatment can be used or heat-shock-treatments may be applied.

Thus, conventional methods are tedious and time consuming, preventing the use of Gram-positive bacteria in a similar wide-spread fashion in biotechnological applications as it is the case for example for *E.coli.*

Furthermore, methods need to be found which simplify the optimization of expression levels of the gene of interest (GOI).

### Description of the Invention

The present invention pertains in one aspect to a method for rapid transformation of at least one gene-of-interest (GOI) in a Gram-positive bacterium by using the following steps:
a. providing a circular DNA with an integrated GOI,
b. obtaining multiplied DNA comprising at least one copy number of the GOI by in-vitro-polymerase-mediated rolling-circle amplification (RCA) from the circular DNA of step a.,
c. transforming the Gram-positive bacterium with the multiplied DNA, by adding the multiplied DNA to a transformation medium, inoculated with the Gram-positive bacterium, and incubating the medium for less than 48h,
d. obtaining clones of the Gram-positive bacterium with at least one copy number of the GOI;
e. (optional) obtaining a gene-product from the Gram-positive bacterium with at least one copy number of the GOI.

One advantage of the present invention is that all of these steps can not only be performed sequentially, but also as a "single-batch", i.e. all steps a.) to d.), or e.), respectively, take place in the same medium (within the transformation medium). Thus, neither an exchange of medium nor any transfer of DNA and/or cells is necessary. This is very time and cost effective.

In another aspect the method of the present invention comprises the combination of DNA ligation methods and rolling circle amplification (e.g. using Φ29 polymerase) in order to enable the omission of the time consuming shuttle strain step. Furthermore, to overcome the limitations of the conventional methods, the new method was also facilitated to integrate one to several-copies of the GOI in a defined gene locus of Gram-positive bacteria, such as *B*. *subtilis.*

Especially the generation of a DNA-product for transformation comprising a range of copy-numbers of the GOI is advantageous for the quick generation of libraries (as will be explained in more detail below).

In a further aspect the method of the present invention refers to a rapid integration of at least one gene-of-interest (GOI) in a defined gene locus of a Gram-positive bacterium by using the following steps:
- providing a circular DNA (plasmid) carrying the GOI integrated in a DNA-sequence homologous to a defined gene locus of the gram-positive bacterium,
- obtaining concatemers of the plasmid by in-vitro polymerase-mediated DNA amplification, resulting in multiplied DNA with at least one copy number of the GOI,
- transforming the gram-positive bacterial-strain with the multiplied DNA, and
- obtaining clones comprising at least one copy of the GOI.

The term "rapid transformation" as used herein, refers to a transformation which enables to obtain clones comprising a gene-of-interest (GOI) within less than 48 hours, preferably less than 24 hours, preferably less than 20 hours, more preferably less than 16 hours, even less than 12 hours, even less than 6 hours. In one embodiment, the new method can be achieved within 12 and 20 hours. In another embodiment, the new method can be achieved within 16 hours.

This is a great advantage as compared to conventional methods, especially such using *E. coli* as "shuttle organism", resulting in transformation protocols which require 2 days or more.

The new combination of the different methods allows also the rapid generation of libraries of Gram-positive bacterium cell factories, e.g. *B. subtilis,* comprising a plurality of copies of the gene of interest (GOI) and preferentially comprising also a heterologous expression of a reporter protein. A full library, with a plurality of Gram positive bacterial clones comprising a range of copy numbers of the GOI, can be created within one round of the steps as described above, resulting in retrieving a clone library within less than 5 days, less than 4 days, preferably within less than 3 days, most preferred within less than 2 days, in some preferred embodiments even less than 24 hours.

The transformation with RCA-multiplied DNA is very effective. The transformation efficacy is in one embodiment between 10² to 10⁸ transformants / µg DNA, in another embodiment between 10³ to 10⁷ transformants / µg DNA, in yet another embodiment between 10⁴ to 10⁶ transformants / µg DNA. In one embodiment the transformation efficacy is at least 10² transformants / µg DNA, at least 10³ transformants / µg DNA, at least 10⁴ transformants / µg DNA, at least 10⁵ transformants / µg DNA, at least 10⁶ transformants / µg DNA, at least 10⁷ transformants / µg, at least 10⁸ transformants / µg DNA or more.

The transformation rate with RCA-multiplied DNA, for example Φ29-multiplied DNA, is at least 10 times, 20 times, 30 times, and up to 50 times, 100 times more efficient as a transfection with conventional DNA-material, such as plasmids or linear DNA generated by PCR- and/or cloning methods.

The transformation of the Gram-positive bacterium with the RCA multiplied DNA can be done by adding less than 10 µg, more preferred less than 5 µg, most preferred less than 1 µg of the multiplied DNA to a transformation medium inoculated with the Gram-positive bacterium and comprising between 0.01% and 1% (wt/vol), more preferred between 0.025% and 0.1% (wt/vol), most preferred about 0.05% (wt/vol) casamino acid (or other amino acid mixtures produced by acid hydrolysis of peptides). In another embodiment genetically modified host cells may be used, which do not necessitate the external supply with amino acid mixtures.

The transformation medium may further comprise at least one substance selected from the group of glucose (C₆H₁₂O₆), tryptophan (C₁₁H₁₂N₂O₂), ammonium iron (III) citrate (C₆H₈O₇ x nFe x nH₃N), monopotassium glutamate (C₅H₈KNO₄), magnesium sulfate (MgSO₄), dipotassium hydrogen phosphate (K₂HPO₄), potassium dihydrogen phosphate (KH₂PO₄), Na₃-citrate, yeast extract, and any combination thereof.

The medium is then incubated for less than 48 hours, less than 24 hours, preferably less than 20 hours, most preferred about 16 hours at a temperature between 20°C and 40°, more preferably between 25°C and 35°C, most preferred at about 30°C and stirred with between 150 to 300 rpm, preferably between 180 and 250 rpm, most preferred at about 200 rpm.

In one embodiment the transformation of the Gram-positive bacterium with the RCA multiplied DNA is done by adding 1 µg of the multiplied DNA to a transformation medium inoculated with the Gram-positive bacterium and comprising about 0.05% (wt/vol) casamino acid. The medium is then incubated for about 16 hours at a temperature at about 30°C and stirred at about 200 rpm.

In another embodiment the Gram-positive bacterium is not pretreated in any way to render it "competent" for a transfection. Such treatments may include chemical treatments (such as treatments with polyethylene glycol or glycine), heat treatments (such as a heat shock), enzymatic treatments (such as lysozyme treatment) and/or use of shuttle cells or systems.

As "gene-of-interest" (GOI) is any gene encompassed which produces a gene-product, such as a RNA , peptide or protein, which in one embodiment does not naturally occur in the Gram-positive host organism. In another embodiment also GOIs are encompassed which do occur naturally in Gram-positive bacteria, but of which only one or a few copy numbers are present in the chromosome of the cell and higher production of the GOI (and the associated gene-product) is desired. Thus, the natural GOI is enhanced by the introduction of additional copy numbers of said GOI.

As such, the present invention also pertains to gene products as well as host cells produced by the new method.

In general, ribonucleic acid (RNA) is a polymeric molecule essential in various biological roles in coding, decoding, regulation and expression of genes. RNA and DNA are nucleic acids. Along with lipids, proteins, and carbohydrates, nucleic acids constitute one of the four major macromolecules essential for all known forms of life. RNA plays an important role both as a structural as well as a signaling molecule within the cell.

Examples for a RNA produced as GOI in the transformed Gram-positive bacterium can be selected from the list comprising messenger RNA (mRNA), ribosomal RNA (rRNA), signal recognition particle RNA (7SL RNA) or (SRP RNA), transfer RNA (tRNA), transfer-messenger RNA (tmRNA), small nuclear RNA (snRNA), small nucleolar RNA (snoRNA), antisense RNA (aRNA, asRNA), CRISPR RNA (crRNA), Long noncoding RNA (IncRNA), microRNA (miRNA), piwi-interacting RNA (piRNA), small interfering RNA (siRNA), short hairpin RNA (shRNA), Trans-acting siRNA (tasiRNA), repeat associated siRNA (rasiRNA), 7SK RNA, enhancer RNA (eRNA), viral RNA, etc.

In general, peptides are selective and efficacious signaling molecules that bind to specific cell surface receptors, such as G protein-coupled receptors (GPCRs) or ion channels, where they trigger intracellular effects. Given their attractive pharmacological profile and intrinsic properties, peptides represent an excellent starting point for the design of novel therapeutics and their specificity has been seen to translate into excellent safety, tolerability, and efficacy profiles in humans. This aspect might also be the primary differentiating factor of peptides compared with traditional small molecules. Furthermore, peptide therapeutics are typically associated with lower production complexity compared with protein-based biopharmaceuticals and, therefore, the production costs are also lower, generally approaching those of small molecules.

Examples for a peptide produced as GOI in the transformed Gram-positive bacterium can be selected from the list comprising hormones, neurotransmitters, growth factors, ion channel ligands, and/or anti-infectives. Well-known examples include insulin, glucagon-like peptide-1 (GLP-1) and other GLP-1 agonists, and/or teduglutide.

In general, proteins are large biomolecules, or macromolecules, consisting of one or more long chains of amino acid residues. Proteins perform a vast array of functions within organisms, including catalyzing metabolic reactions, DNA replication, responding to stimuli, providing structure to cells, and organisms, and transporting molecules from one location to another. Proteins differ from one another primarily in their sequence of amino acids, which is dictated by the nucleotide sequence of their genes, and which usually results in protein folding into a specific 3D structure that determines its activity.

Examples for a protein produced as GOI in the transformed Gram-positive bacterium can be selected from the list comprising ADAM, actins, adaptor-related protein complexes, ADP-ribosylation factor, albumins, allergens, alpha-2-hs-glycoproteins, anaplasma related proteins, endothelial cell-specific molecule 1, angiogenins, proteins comprising an ankyrin repeat domain, annexins, anterior gradient proteins, ASF1 anti-silencing function 1, mitochondrial ATP synthases, ATPases, autophagy related proteins, Bartonella H. related proteins, B cell lymphoma associated proteins, B9 protein, Babesia Microti related proteins, Baculoviral lap repeat-containing proteins, BATFs, basic transcription factors, BAX, beta 2 microglobulin, BIDs, biglycans, bromodomain containing proteins, bridging integrators, cadherins, calbindins, calcium binding proteins, calcyphosine, calmodulin, calpain, calponin, calreticulin, calumenins, Candida Albicans related proteins, canopy FGF signaling regulators, capping proteins, CEA, troponins, caspase recruitment domain family related proteins, CEBP, CCR4-not transcription complex, cell division cycle proteins, cellular retinoic acid binding protein, centrins, centromere proteins, charged multivesicular body proteins, chloride intracellular channels, chromatin modifying proteins, chromobox comprising proteins, chromogranins, chromosome open reading frame proteins, clathrin, coagulation factors, cofilin, coiled-coil domain comprising proteins, collagen, comm domain containing, complement component, COP9 signalosome, c-reactive protein. crystallin, C-type lectin domain, cue domain containing, cystatin, cysteine-rich-proteins, cysteine-rich secretory proteins, cytochrome, cytohesin, cytokeratin, DCUN1D, dead box protein, decorin, density lipoproteins, developmental pluripotency associated proteins, dickkopf-related proteins, Digeorge syndrome critical region, DNA-damage proteins, dynactin, dynein light chain comprising proteins, dysbindin, ectodysplasin, elongator acetyltransferase complex, endothelial cell-specific molecule 1, proteins found in the endoplasmic reticulum, EPH receptors, ephrin, eukaryotic translation initiation factors, ERCC, exosome components, FABP, f-box proteins, ferritin, fibrinogen, fibronectin type iii, domain containing, four and a half lim, proteins of the fragile histidine triad, G antigens, GABA(a) receptor-associated proteins, gastrokines, GDP dissociation inhibitors, general transcription factors, GIPC PDZ domain comprising proteins, gliadin, glycophorin, glycoprotein NMB, glypican, gremlin, proteins of the GTPase IMAP family, GTP-binding proteins, guanine nucleotide binding proteins, hairy and enhancer of split, haptoglobin, heat shock proteins, hematological and neurological expressed proteins, hemoglobins, hemopexins, HNRNPs, proteins belonging to the high-mobility group, hints, homeobox comprising proteins, homer homologs, hypoxia-inducible factors, Ig heavy chain regions, Ig variable chain regions, IMPAD1, inhibitors of DNA binding, proteins belonging to the inhibitors of growth family, integrins, intercellular adhesion molecules, karyopherin, junctional adhesion molecules, proteins comprised in killer cells, killer cell lectin-like receptors, kirsten rat sarcoma viral oncogenes, kruppel-like factors, lactoferrins, left-right determination factors, leukocyte cell derived chemotaxins, leukocyte-associated ig-like receptors, LIN proteins, LBPs, listeriolysins, LRG1, lymphocyte antigens, lysosomal-associated membrane proteins, MAD2, mago-nashi homologs, HLAs, maltose binding proteins, proteins belonging to the mediator complex, proteins belonging to the melanoma antigen family a, proteins belonging to the members of the ras oncogene family, mesoderm development candidates, met proto-oncogenes, methylmalonic aciduria related proteins, MHC class I chain-related genes, microfibrillar associated proteins, microtubule-associated proteins, mitochondrial ribosomal proteins, mitochondrial transcription factors, MOB1, MPS one binder kinase activators, mortality factors, AG85, myelin basic proteins, myelin oligodendrocyte glycoproteins, myoglobins, myosin light chain, myxovirus related proteins, nanogs, NCK adaptor proteins, nescient helix loop helix comprising proteins, nectins, neuronal calcium sensors, neutrophil cytosolic factors, NFKB inhibitos, NHP2, n-MYC downstream regulated proteins, proteins comprised in non-metastatic cells, NTF2-like export factors, nucleobindins, npms, nucleopurins, nucleosome assembly proteins, orosomucoid, TNF, outer membrane proteins, p53, paired box proteins, parkinson disease proteins, parvalbumins, PCNAS, PDZ domain containing proteins, pentraxin, peroxisomal biogenesis factors, PHD finger proteins, phosducin-like proteins, phosphatases and tensins, PITPNs, phospholipid scramblases, PIH1 domain containing proteins, PIM oncogenes, polymerases (RNA) (DNA directed), KCTDs, POU class proteins, prefoldins, pregnancy specific beta-1-glycoproteins, prion proteins, processing proteins of precursor proteins, profilins, proteins associated with programmed cell death, prohibitins, proprotein convertases subtilisin/kexin, protein C-ETS, protein phosphatases, protein-A, A/G & G, prothymosins, RAD51, RASSF, RAS-related c3 botulinum toxin substrates, receptor activity-modifying proteins, regenerating islet-derived proteins, regulators of calcineurin, regulators of g-protein signaling, related ras viral (R-RAS) oncogenes, relaxins, renins, reticulocalbins, retinoblastoma related proteins, retinoic acid early transcript proteins, retinoic acid receptor responder proteins, rho family GTPases, rho GDP dissociation inhibitors, ribosomal proteins, ring finger proteins, RNA binding motif proteins, r-spondins, RWD domain containing proteins, sclerostins, secretagogins, secreted frizzled-related proteins, secretoglobins, secretogranins, selectins, selenoproteins, septins, serglycins, serine peptidase inhibitors, serpins, serta domain containings, SH2 domain comprising proteins, siglecs, SH3 domain comprising proteins, signal recognition particles, signal sequence receptors, signal-regulatory proteins, single-stranded DNA binding proteins, sirtuins, six homeobox comprising proteins, proteins of the slam family, SMADs, SNRPs, proteins belonging to the solute carrier family, sorting nexins, ran binding proteins, SRAGE, SPSBs, SPARCs, sry (sex determining region y)-box comprising proteins, stathmins, streptavidin proteins, STIPs, SDHAFs, superoxide dismutases, surfeits, synaptobrevins, synaptosomal associated proteins, synaptotagmins, syndecans, synovial sarcoma related proteins, x breakpoint proteins, syntaxins, synucleins, tachykinins, TAR DNA proteins, proteins belonging to the tbc1 domain family, TCLs, TTCs, Toll like receptors, THAP domain comprising proteins, thioredoxins, tigars, tissue factor pathway inhibitors, TNF receptor-associated factors, trafficking protein particle complexes, transcription elongation factors, transferrins, transforming growth factor beta induced proteins, transgelins, translocase proteins of outer mitochondrial membrane, proteins of the triggering receptor expressed on myeloid cells, proteins comprising a tripartite motif, tropomyosins, proteins of the trove domain family, trypsins, tubulin folding cofactors, U6 small nuclear RNA, tubulin gamma, ubiquinol-cytochrome c reductases, ubiquitins, UCHL1, UL16 binding proteins, uroplakins, proteins related to vacuolar protein sorting, vascular cell adhesion molecules, V-CRK sarcoma virus CT10 related proteins, vimentins, visinin-like proteins, MAFs, v-ral simian leukemia viral oncogenes, WAP FOUR-disulfide core domain comprising proteins, *Y. enterocolitica* related proteins, zinc finger comprising proteins, as well as fragments and combinations thereof.

In one embodiment for example viral RNA may be produced in order to provide substrate for the development of vaccines and/or analytic and/or therapeutic antibodies. In one embodiment RNA of coronaviridae, e.g. SARS-CoV-2, is produced.

In a further embodiment the method of the present invention may be used to produce a vaccine within the host strain, e.g. in form of a RNA, peptide or protein which resembles the antigen of pathogen against the vaccination shall take place. In one embodiment the method of the present invention may be used for example to produce a vaccine against a SARS-CoV-2 infection, e.g. by producing part of the SARS-CoV-2 glycoprotein S, preferable the S1-subunit or RBD-domain of said SARS-CoV-2 virus.

In yet another embodiment the method of the present invention may be used to produce antigen-binding proteins, such as for example analytical and/or therapeutic immunoglobulins, such as IgG, IgM, IgA, IgD, IgE, etc.

It is important to note that the rapid transformation method of the present invention can be used to produce both cells expressing the GOI "stably" as well as "transiently", based on the composition of the transfected DNA.

If the GOI is to be "stably expressed" the aim is to integrate the GOI into the host's genome. Cell-lines expressing the GOI stably possess the advantage of easy propagation and a more reliable expression profile.

In some embodiments in which as stable expression is preferred the transfected DNA comprises sequences homologous to a target within the host's chromosome. Additional factors may be used to facilitate an integration into the host chromosome even further (i.e. a CRISPR/Cas-system).

Cell-lines expressing the GOI transiently, i.e. in form of a plasmid which is not integrated into the host's chromosome, may be used with more ease in order to combine the expression of different GOIs in one host cell. Thus, in embodiments in which a transient expression is preferred sequences homologous to a target within the host's chromosome are not present.

Transiently expressing cell lines may also produce the gene-product of the GOI in higher abundance, especially when provided with a strong promoter and/or a replication function enabling copy number higher than one.

Thus, depending on the application, both expression systems are encompassed within embodiments of this invention.

In both cases additional modifications may be present in the transfected DNA, such as a strong promoter, a reporter gene, and the like.

In one embodiment the present invention pertains to Gram-positive bacteria, such as *B*. *subtilis, B. licheniformis, B. megaterium, B. pumilus, B. clausii, B. halodurans, Paenibacillus polymyxae* and *Paenibacillus riograndensis*; or any combination thereof.

In another embodiment a member of the *B*. *subtilis group* is preferred, such as *Bacillus amyloliquefaciens, Bacillus licheniformis, Bacillus subtilis, Bacillus atrophaeus, Bacillus vallismortis, Bacillus sonorensis* and *Bacillus mojavensis* subgroup.

In yet another embodiment *Bacillus subtilis* is even more preferred for the inventive transformation method.

In another embodiment the Gram-positive bacterium is selected from the phylus of *firmicutes,* such as the group comprising *B*. *subtilis, B. licheniformis, B. megaterium, B. pumilus, B. clausii, B, halodurans, B. amyloliquefaciens, B. atrophaeus, B. vallismortis, B. sonorensis* and *B*. *mojavensis subgroup; and any combination thereof.*

In another embodiment the Gram-positive bacterium is selected from the genus *Paenibacillus* such as *Paenibacillus polymyxae* and *Paenibacillus riograndensis; and any combination thereof.*

The term "in-vitro-polymerase-mediated rolling-circle amplification (RCA)" as used herein, describes a process of unidirectional nucleic acid replication that can rapidly synthesize multiple copies of circular molecules of DNA or RNA, such as plasmids, the chromosomes of bacteriophages, and the circular RNA chromosome of viroids. Some eukaryotic viruses also replicate their DNA or RNA via the rolling circle mechanism.

As a simplified version of natural rolling circle replication, an isothermal DNA amplification technique, rolling circle amplification (RCA) was developed.

The polymerases used in RCA are Φ29 (Phi29), Bst, and Vent exo-DNA polymerase for DNA amplification.

Since Φ29 DNA polymerase has the best processivity and strand displacement ability among all aforementioned polymerases, it is preferred in this invention. However, depending on the specific problem, also the other polymerase could be applied in other embodiments.

Different from polymerase chain reaction (PCR), RCA can be conducted at a constant temperature (for example at room temperature of 25°C, at 30°C or up to 37°C, in other embodiments at a temperature between 4°C - 45°C) in both free solution and on top of immobilized targets (solid phase amplification).

There are typically three steps involved in a DNA RCA reaction:
- Circular template ligation, which can be conducted via template mediated enzymatic ligation (e.g., T4 DNA ligase) or template-free ligation using special DNA ligases (i.e., CircLigase).
- Primer-induced single-strand DNA elongation. Multiple primers can be employed to hybridize with the same circle. As a result, multiple amplification events can be initiated, producing multiple RCA products ("Multiprimed RCA"). A linear RCA product can be converted into multiple circles using restriction enzyme digestion followed by template mediated enzymatic ligation.
- Amplification product detection and visualization, which is most commonly conducted through fluorescent detection, with fluorophore-conjugated dNTP, fluorophore-tethered complementary or fluorescently-labeled molecular beacons. In addition to the fluorescent approaches, gel electrophoresis is also widely used for the detection of RCA product.

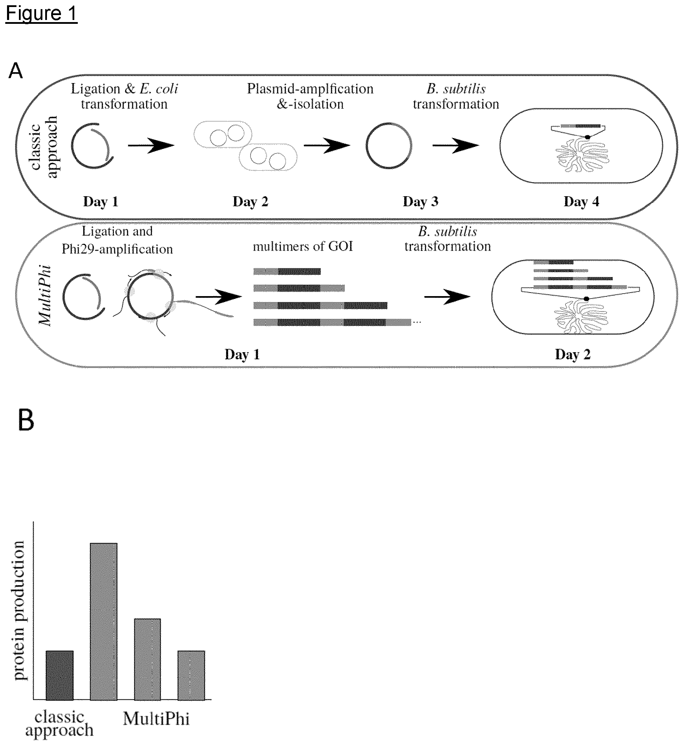

In one embodiment of the new method, the RCA-DNA amplification step by the Φ29 polymerase is added to the classic approach for gene integrations in *B*. *subtilis* (see Fig.1). The polymerase Φ 29 and random primer hexamers may be used to amplify the desired circular DNA with the GOI.

It is a surprising finding of the present invention, that RCA-multiplied DNA, especially Φ29-multiplied DNA, can be transfected directly into a Gram-positive bacterial-strain without the necessity to use a shuttle strain or the necessity to render the host strain "competent for infection" by a pretreatment with chemical and/or physical means.

Without being bound to any theory, the surprisingly strongly improved uptake of RCA-amplified DNA may be due to the formation of fresh DNA which is not physically damaged such as with nicks. Furthermore, no modifications such as methylation patterns are present which might induce destruction through endonucleases in some hosts. The DNA is produced in linear form which is the form naturally competent organisms require for uptake.

The DNA-substrate for the RCA-reaction is preferably circular. The RCA-multiplied DNA as substrate for the transformation of the host cell is preferably linear. However, in some embodiments it may be present in circular form.

The RCA-multiplied DNA is then subsequently transformed into the Gram positive bacterium, for example a *B. subtilis*-strain, such as *B. subtilis* PY79 or *Bacillus licheniformis* strains such as *B. licheniformis NW3.*

The rolling-circle amplification (RCA) necessitates a circular DNA as substrate. Thus, either linear DNA is produced by conventional methods, for example by artificial gene synthesis, cloning techniques, polymerase-chain-reaction (PCR) and/or extraction methods from natural sources, e.g. an organism (such as yeast, E. coli, zebrafish, mouse, etc.) or a microbiome, or a combination thereof. If the DNA is linear, it can be made circular by conventional methods known to the skilled person, such as for example ligation techniques. In other embodiments the DNA is already produced in circular form, e.g. as a plasmid by a host organism.

The term "multiplied DNA" refers to any DNA which was multiplied with a rolling circle amplification (RCA). It is therefore used herein for any product of the RCA-process. The multiplied DNA as a result of the RCA-reaction may be present in linear or circular form.

The multiplied DNA may be produced in one embodiment as a single copy DNA of the original DNA which was used as substrate for the RCA-reaction (often with just one copy of the GOI and the reporter gene), in other embodiments as a concatemer (often with a plurality of copies of the GOI combined with either one copy, or a plurality of reporter genes).

The term "concatemer" is used herein for a long continuous DNA molecule that contains at least two, in some embodiments at least three, at least five, or more multiple copies of the same DNA sequence linked in series.

Concatemers are frequently the result of a rolling circle amplification (RCA), however, they occur also natural in the late stage of bacterial infection by phages and during viral infections.

As an example, if the genes in a substrate-DNA are arranged ABC, then in a concatemer the genes would be ABCABCABCABC and so on.

The gene copy numbers can be determined by quantitative real-time PCR of the isolated chromosomal DNA (see example section for details). Also the genomic stability of multiple integrations can be investigated by sub-cultivations with and without selection pressure (see example section for details).

One advantage of having multiple copies of the GOI is the higher production rate of the gene-product of such a GOI.

Furthermore, an unequal distribution of GOI-copies per host cell allows to create a clone library from which the clone with the best expression rate due to the ideal copy number and ideal integration locus (in case of a stable expression) can be picked depending on the circumstances, such as medium composition, growth conditions and the like.

Especially proteins which are toxic to the host or difficult to express may necessitate smaller copy numbers and/or a copy number adapted to the conditions which allow a better survival of the host cell and/or correct folding of the protein. For example proteins with a complex tertiary structure may necessitate growth at reduced temperatures or in media deprived of certain factors.

On the other hand, high-throughput expression may be better with cells comprising high copy numbers of the GOI as compared to cells with low GOI copy numbers but strong promotors.

In any case a library representing a whole range of GOI copy numbers, integration loci and/or transient expressed GOIs may be generated in a short amount of time by the new methods of this application. For selection of the best clone the heterologous expression of at least one reporter protein (e.g. a fluorescence-molecule and/or an antibiotics-resistance and/or peptide epitope, e.g. His-tag) is preferred besides the GOI.

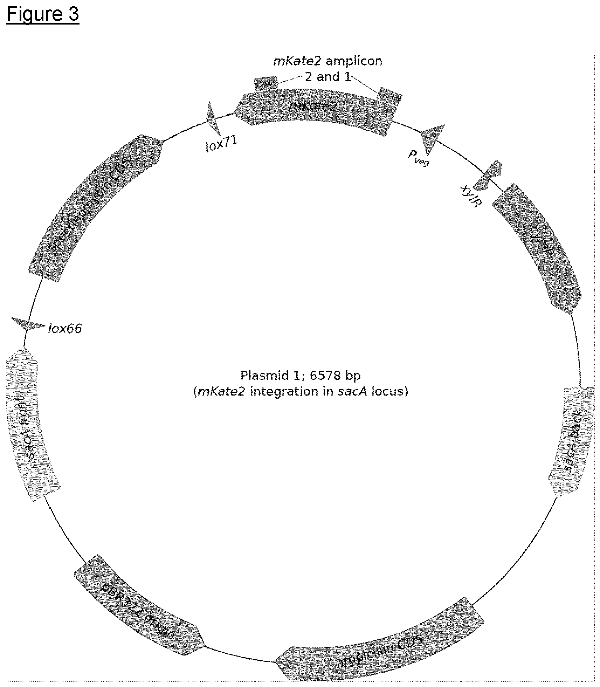

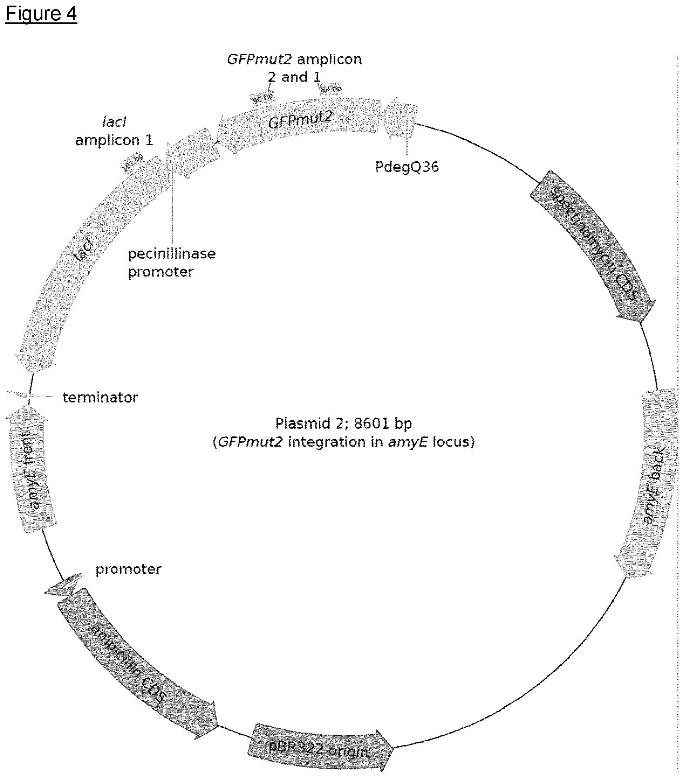

The following SEQ IDs are provided with this application:
SEQ ID No. 1 - Describes the GFPmut2-amplicon 1 in "forward direction" (cf. also Figure 3).
SEQ ID No. 2 - Describes the GFPmut2-amplicon 1 in "reverse direction" (cf. also Figure 3).
SEQ ID No. 3 - Describes the GFPmut2-amplicon 2 in "forward direction" (cf. also Figure 3).
SEQ ID No. 4 - Describes the GFPmut2-amplicon 2 in "reverse direction" (cf. also Figure 3).
SEQ ID No. 5 - Describes the MKate2-amplicon 1 in "forward direction" (cf. also Figure 4).
SEQ ID No. 6 - Describes the MKate2-amplicon 1 in "reverse direction" (cf. also Figure 4).
SEQ ID No. 7 - Describes the MKate2-amplicon 2 in "forward direction" (cf. also Figure 4).
SEQ ID No. 8 - Describes the MKate2-amplicon 2 in "reverse direction" (cf. also Figure 4).

### Description of Figures

- Figure 1 -: **A.** Graphical abstract comparing the state-of-the-art *B*. *subtilis* transformation process to the inventive method. The classic approach takes up to four days and only results in the integration of one copy of the gene of interest (GOI). The inventive method ("MultiPhi") uses Phi29-based rolling-circle amplification (RCA) to amplify circularized DNA assemblies instead of *E. coli* based plasmid selection and amplification. RCA results in multimers of the GOI which can be directly transformed into *B. subtilis.* **B.** The resulting inventive clones each contain different copy numbers of the GOI, resulting in a broad spectrum of produced protein on the second day.

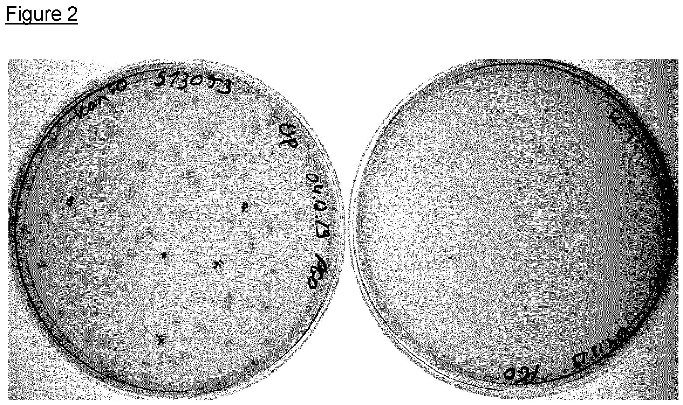

- Figure 2 -: Transformation plates of *B*. *subtilis* using RCA polymerase (left) and standard protocol (right).
- Figure 3 -: Example DNA construct for transfection according to the inventive method. DNA will integrate into target locus (sacA) of host cell after transfection.
- Figure 4 -: Example DNA construct for transfection according to the inventive method. DNA will integrate into target locus (amyE) of host cell after transfection.

### Examples

### Example 1. Production of Plasmids

Integration plasmids were assembled by the ligase cycling reaction with the purpose to integrate in *B. subtilis* PY79 by homologous recombination. The heterologous gene mKate2 was integrated as a single copy in the *sacA*-locus via plasmid 1 (Figure 3). Afterwards, this strain was used as target for the insertion of GFPmut2 via plasmid 2 (Figure 4) and the Φ29-amplified product.

### Example 2. Φ29 amplification of plasmids

To obtain concatemers of the plasmid, the multiply-primed rolling circle amplification was applied (RCA). For the amplification, 1 µL of isolated plasmid (50 ng) was mixed with 9 µL sample buffer of the GenomiPhiTM V2 DNA Amplification Kit (GE Healthcare UK Limited, Buckinghamshire, UK). After 3 min at 95°C and 1 min on ice, 9 µL reaction buffer and 1 µL Φ29-enzyme mix was added followed by an incubation at 30°C for 1.5 h and the inactivation at 65°C for 10 min.

Subsequently, the total volume of the multiplied DNA was used for the transformation of *B*. *subtilis* without any further processing.

### Example 3. Chromosomal integration in Bacillus subtilis

For the chromosomal integrations via the natural competence of *B*. *subtilis sp.,* the *amyE* locus of the non-sporulating strain PY79 was used. Prior to the GFPmut2 integration, a single copy of mKate2 was inserted in the *sacA* locus for the normalization of the GFP signal in the growth measurements and qPCR analytics.

For the transformation 20 µL of plasmid-DNA (1 µg) or 20 µL Φ29-multiplied DNA was added to 1mL of inoculated transformation medium with 0.05%m/v casamino acids (Sigma Aldrich, St. Louis, MO, USA).

After an incubation for 16h at 30°C and 200 rpm, the suspensions were plated on 1%m/v agar with 100 µg/mL spectinomycin. The transformation efficiency (CFU µg⁻¹ DNA) was calculated by dividing the counted CFUs per agar plate by the mass of plasmid DNA used for the transformation.

### Example 4. Growth curve and fluorescence measurement

The incubation, growth curve and fluorescence intensity measurements (Figure A4) were automatically performed at 30°C by a robotic platform and a microplate reader (CLARIOstar^{®} Plus, BMG Labtech Ortenberg, Germany). The subcultivations shown in Figure 4 were performed by using the same microplate reader and two incubators at 30/37°C and 200 rpm for the cultivation.

For all measurements, flat-bottom 96-multi-well plates (Cellstar^{®}, Greiner Bio-OneTM GmbH, Frickenhausen, Germany) and a total volume of 200 µL per well were utilized to measure the optical density (wavelength: 600nm) and the fluorescence intensities (GFPmut2: extinction/emission at 485/520nm, mKate2: 575/620nm). The fluorescence was measured from top and by using a fixed gain within one experiment. For plotting the results, R (v. 3.6.1) and RStudio (v. 1.2.1335, [34]) were applied.

### Example 5. Chromosomal DNA isolation and qPCR

Chromosomal DNA of B. subtilis strains was isolated by using the High Pure PCR Template Preparation Kit (Roche Diagnostics GmbH, Mannheim, Germany). For the isolation, 200 µL of an overnight culture were utilized. To determine the gene copy number of the integrated GFPmut2, the isolated DNA was used as template for the qPCR. For this, the SYBRTM Green PCR Master Mix of Thermo Fisher Scientific (Applied BiosystemsTM, MA, USA) and the StepOnePlusTM Real-Time PCR System was applied to determine DNA copy numbers. Two amplicons of mKate2 and GFPmut2 were amplified (see Figures 3 and 4). The sequences of the amplification primers are shown in the sequence protocol.

### Example 6: Transformation efficacy

To evaluate the transformation efficiency of *B*. *subtilis* using high copy plasmids treated with RCA polymerase, three independent experiments were conducted.

In each case, 1 mL of *B*. *subtilis* transformation culture was transformed with 2.5 ng of high copy plasmid treated with RCA polymerase. As a control, 1 mL transformation culture of the same strain was transformed with 1 µg of the respective plasmid. All transformations were done under the same conditions. Transformation efficiency was assessed by counting colony forming units (CFU). Results are presented in Table 1.

**Table 1. Transformation efficiency of high copy plasmids in different B. subtilis strains.**

| Experiment | Method | Plasmid | CFU/1 ml Transformation |
|---|---|---|---|
| Bs02029+p02030 | RCA-Polymerase treatment | 2.5 ng | 101 |
| | Without treatment (control) | 1 µg | 2 |
| Bs95001+p95002 | RCA-Polymerase treatment | 2.5 ng | 876 |
| | Without treatment (control) | 1 µg | 1 |
| Bs95001+p95003 | RCA-Polymerase treatment | 2.5 ng | 536 |
| | Without treatment (control) | 1 µg | 5 |

## Claims

1. A method for rapid transformation of at least one gene-of-interest (GOI) in a Gram-positive bacterium by using the following steps:
a. providing a circular DNA with an integrated GOI,
b. obtaining multiplied DNA comprising at least one copy number of the GOI by in-vitro-polymerase-mediated rolling-circle amplification (RCA) from the circular DNA of step a.,
c. transforming the Gram-positive bacterium with the multiplied DNA, by adding the multiplied DNA to a transformation medium, inoculated with the Gram-positive bacterium, and incubating the medium for less than 48h,
d. obtaining clones of the Gram-positive bacterium with at least one copy number of the GOI;
e. (optional) obtaining a gene-product from the Gram-positive bacterium with at least one copy number of the GOI.

2. The method according to claim 1, wherein the polymerase is selected from the group comprising *Φ29 (Phi29), Bst,* and/or *Vent exo*-DNA polymerase.

3. The method according to any of claims 1 or 2, wherein at least two copy numbers of the GOI are in the multiplied DNA and wherein the clones obtained comprise different copy numbers of the GOI, resulting in a broad spectrum of produced protein concentrations.

4. The method according to any of the previous claims, wherein the at least one copy of the GOI is stably expressed in the target bacterium.

5. The method according to any of the previous claims, wherein the at least one copy of the GOI is transiently expressed in the target bacterium.

6. The method according to any of the previous claims, wherein the Gram-positive bacterium is selected from the phylus of *firmicutes* selected from the group comprising *B. subtilis, B. licheniformis, B. megaterium, B. pumilus, B. clausii, B, halodurans, B. amyloliquefaciens, B. atrophaeus, B. vallismortis, B. sonorensis* and *B*. *mojavensis subgroup; and any combination thereof.*

7. The method according to any of the previous claims, wherein the Gram-positive bacterium is selected from the genus *Paenibacillus* selected from the group comprising *Paenibacillus polymyxae* and *Paenibacillus riograndensis; and any combination thereof.*

8. The method according to any of the previous claims, wherein the transformation efficacy is at least 20 fold increased as compared to a transformation method with a PCR-amplified DNA in said bacterium.

9. The method according to any of the previous claims, wherein 1 µg DNA / ml medium is sufficient for a successful transformation.

10. The method according to any of the previous claims, wherein in step c. the medium is incubated less than 20 hours.

11. The method according to any of the previous claims, wherein the Gram-positive bacterium comprises additionally at least one reporter protein.

12. The method according to any of the previous claims, wherein an amino acid hydrolysate is present in the transformation medium of step c.

13. A gene product produced by any of the methods of the claims 1 to 12.

14. Gram-positive bacterium produced by any of the methods of the claims 1 to 12.

15. Use of the method according to claims 1 to 12, the gene product of claim 13, or the Gram-positive bacterium of claim 14 for the production of a nucleic acid, protein or peptide.
